# EUROPÄISCHE PATENTSCHRIFT

(11) **EP 2 593 031 B1**
(45) Veröffentlichungstag und Bekanntmachung des Hinweises auf die Patenterteilung: **02.11.2016**
(21) Anmeldenummer: 11779211.9
(22) Anmeldetag: 14.07.2011
(51) Int. Cl.: A61B 5/107, A61B 34/20

(54) **OPTISCHES VERFAHREN ZUR ERMITTLUNG EINER BEINLÄNGENÄNDERUNG**
OPTICAL METHOD FOR DETERMINING THE CHANGE OF LENGTH OF A LEG
MÉTHODE OPTIQUE POUR DÉTERMINER UN CHANGEMENT DE LONGUEUR D'UNE JAMBE

(30) Priorität: 15.07.2010 DE 102010027336
(43) Veröffentlichungstag der Anmeldung: 22.05.2013
(73) Patentinhaber: Naviswiss AG, 4242 Laufen (CH)
(72) Erfinder: KNOBEL, Bruno, 4242 Laufen (CH); FINDEISEN, Charles, 5430 Wettingen (CH)
(74) Vertreter: Castell, Klaus
(86) Internationale Anmeldenummer: PCT/IB2011/002316
(87) Internationale Veröffentlichungsnummer: WO 2012/007841

(56) Entgegenhaltungen:
- EP-A1- 1 563 810
- EP-A2- 0 310 901
- US-A- 4 631 676
- US-A- 5 249 581
- US-A1- 2004 106 861

## Beschreibung

Die Erfindung betrifft ein Verfahren zum Betreiben eines optischen, stereometrischen Mess-Systems zur Ermittlung einer Beinlängenänderung, bei dem auf der Haut eines ausgerichteten Patienten Markierungen über den beiden vorstehenden Beckenknochen (anterior superior iliac spine) und an den beiden Fußgelenken angebracht und bei dem für eine erste und eine zweite Messung am ausgerichteten Patienten mit mindestens zwei ausgerichteten Kameras die Raumkoordinaten dieser Markierungen sowie deren Beziehungen untereinander in Form von Strecken und Winkeln ermittelt werden.

Heute werden pro Jahr mehr als 1.3 Millionen (mit zweistelliger Zuwachsrate) künstliche Hüftgelenke bei Patienten eingesetzt. Bei diesem Eingriff wird angestrebt, die ursprüngliche oder biomechanisch optimale Beinlänge nach Einsetzung des Gelenkersatzes wieder zu erreichen. Je nach Geschick und Erfahrung des Orthopäden und je nach Anatomie des Patienten kann dieses Ziel erreicht werden. Dabei orientiert sich der Orthopäde beispielsweise an charakteristischen Knochenteilen wie beispielsweise an Femur und Pelvis oder an spezifischen Stellen der Fußgelenke.

US4631676 offenbart ein stereoskopisches Messsystem bei dem Marker auf der Haut über dem Becken und einem der Fussknöchel einer Person angebracht werden.

Bei den meisten Eingriffen dieser Art steht allerdings keine geeignete Vorrichtung zum Messen von Beinlängen zu Verfügung. Damit basieren Details des Eingriffs auf der visuellen Beurteilung des Orthopäden.

Mit den heute zur Verfügung stehenden Navigationssystemen könnte diese Aufgabe grundsätzlich gelöst werden. Jedoch ist deren Einsatz und Handhabung aufwendig und ergonomisch wenig sinnvoll. Des Weiteren sind sehr viele OP- Räume damit nicht ausgestattet.

Therapeuten und Chiropraktiker protokollieren beim Patienten dessen Zustand und/oder den Einfluss ihrer Behandlung unter anderem mittels Fotos und/oder Messmitteln wie Längen- oder Winkelmaß.

Als Anwender werden im folgenden Chirurgen, Orthopäden, Chiropraktiker, Therapeuten und andere Personen mit entsprechenden medizinischen Kenntnissen bezeichnet. Anwender können aber auch Mitarbeiter sein, die für eine medizinisch ausgebildete Person die Messungen vornehmen.

Der Erfindung liegt die Aufgabe zugrunde, ein Verfahren bereitzustellen, mit dem Lebewesen schnell und einfach vermessen werden können.

Diese Aufgabe wird mit einem gattungsgemäßen Verfahren gelöst, bei dem die Ermittlung der Beinlängenänderung den Vergleich der Strecken und Winkel sowie die Spiegelsymmetrie der Positionen der Markierungen beinhaltet.

Vorteilhafte Ausführungsvarianten sind Gegenstand der Unteransprüche.

Die Erfindung geht vom Grundgedanken aus, dass dem Anwender einfache und mit wenig Aufwand zu handhabende Verfahren mit dem Messsystem zur Verfügung gestellt werden. Damit kann der Anwender die ihn unterstützenden Messaufgaben schnell und zuverlässig durchführen. Die Resultate der Messungen dienen dem Anwender zur Beurteilung der Situation, als Hilfe bei der Durchführung der nächsten Arbeitsschritte oder zur Dokumentieren.

Eine Referenz ist in diesem Sinne entweder ein Datensatz einer früheren Messung, der einen früheren Zustand beschreibt, oder ein Soll-Datensatz, der ein anzustrebendes Ergebnis oder einen Zwischenzustand beschreibt.

Im Folgenden wird unter einem optischen, stereometrischen Messsystem (kurz: Messsystem) die Kombination aus einem Kamerasystem und Referenzmarkierungen (kurz Markierungen) verstanden.

Ein solches Messsystem ist Stand der Technik. Im Folgenden werden weitere Eigenschaften des Messsystems vorgestellt um die Grundgedanken der Erfindung des Verfahrens zu erläutern.

Das Kamerasystem besteht aus mit mindestens zwei stereometrisch ausgerichteten Kameras und Mitteln für die Analyse der Bilddaten und die Ausgabe der Resultate.

Das Kamerasystem erfasst die Markierungen, analysiert die Bilddaten mittels bekannter Methoden für die Kamerabildanalyse, bestimmt mittels bekannten photogrammetrischen Methoden die räumliche Lage der Markierungen in einem Koordinatensystem des Kamerasystems und stellt dem Anwender die gewünschten Abstände zwischen Markierungen und/oder Winkel zwischen Abständen zur Verfügung.

Das Kamerasystem kann in die Einrichtung des Behandlungsraums fest integriert sein. Alternativ kann das Kamerasystem an einem mobilen Stativ angebracht sein. Das Kamerasystem kann so ausgerichtet sein, dass beispielsweise eine Koordinatenachse des Kamerakoordinatensystems parallel zum Lot ist. Damit können beispielsweise die Abweichungen von zwei Markierungen zur Horizontalen quantitativ gemessen werden.

Alternativ kann sich das nicht ausgerichtete Kamerasystem mittels bekannten Methoden für Kamerabildanalyse an Objekten orientieren. Diese Objekte können beispielsweise mit Markierungen versehene geneigte Referenzebenen sein.

Eine weitere Möglichkeit ist, dass das Kamerasystem mobil und manuell gehalten bedient wird.

Die Markierungen werden vom Anwender an oder in der Nähe von biomechanisch optimalen und/oder anatomisch geeigneten Stellen des Patienten oder an Objekten angebracht. Die Markierungen können beispielsweise kleine mit einem Filzstift auf der Haut aufgezeichnete Kreise, Kreuze oder Linien unmittelbar oberhalb von oder zwischen markanten Knochenpartien sein. Eine weitere Möglichkeit für Markierungen sind entfernbare Tätowierungen auf der Haut oder klebende Markierungen mit oder ohne aus der Fotogrammmetrie bekannte Kodierung für die Identifikation der Markierungen.

Markierungen auf der Haut können sich durch eine Behandlung des Lebewesens oder während der Behandlung relativ zur markanten Knochenpartie verschieben. Sie können auch während der Behandlungsdauer ausgelöscht werden. Der Anwender kann in einem solchen Fall die Markierungen erneuern oder ergänzen.

In der Praxis wird mit einem eingangs beschriebenen Messgerät gearbeitet, wobei der Anwender an geeigneten Stellen Markierungen am Patienten anbringt und mit dem Messgerät die Raumkoordinaten der Markierungen sowie deren Beziehungen untereinander in Form von Strecken oder Winkeln ermittelt.

Die Messungen können zur quantitativen Bestimmung eines Zustandes für eine Diagnose dienen. Die Messungen können auch der quantitativen Bestimmung eines Zustandes vor und nach bestimmten therapeutischen oder chirurgischen Eingriffen dienen.

Die Resultate der Messungen können vom Anwender für die Dokumentation verwendet werden.

Der allgemeine Erfindungsgedanke besteht in einem Messverfahren zur Begleitung von medizinischen Eingriffen und therapeutischen Maßnahmen unter Verwendung eines optischen Kamerasystems mit mindestens zwei Kameras in Stereoanordnung und mindestens zwei an geeigneten Stellen eines Lebewesens angebrachten Markierungen, bei dem diese Markierungen zumindest vor und nach einer Behandlung des Lebewesens mit dem optischen Kamerasystem erfasst werden, die dreidimensionalen Koordinaten dieser Muster im Kamerakoordinatensystem ermittelt und in geeigneter Form dem Anwender zur Verfügung gestellt werden.

Vorzugsweise beinhaltet das Verfahren die Verwendung eines fix installierten Kamerasystems. Eine weitere Ausgestaltung der Erfindung sieht vor, dass mobile Kamerasysteme verwendet werden.

Eine besonders vorteilhafte Ausgestaltung sieht vor, dass das Verfahren eine Hüftoperation begleitet. Weitere Anwendungsbereiche sind Wirbelsäulenoperationen, chiropraktische oder therapeutische Behandlungen und die Messung der Beweglichkeit eines Körperteils.

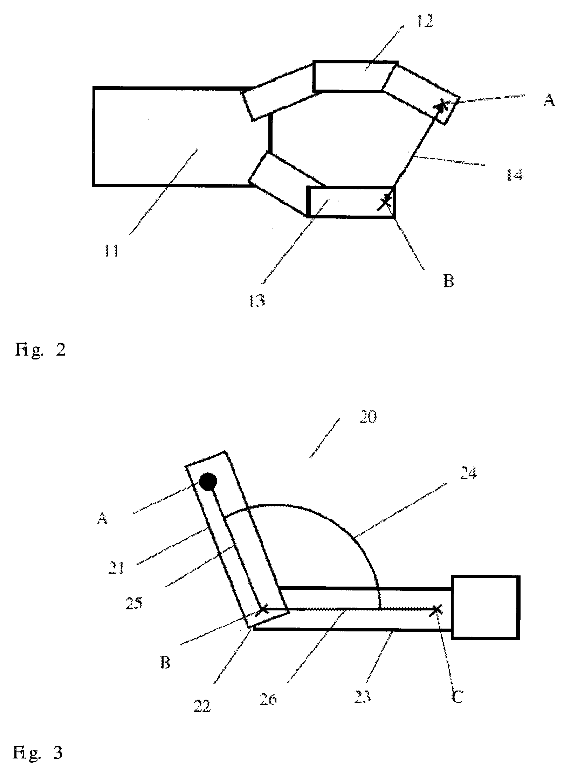

Die Erfindung wird nachstehend ohne Beschränkung des allgemeinen Erfindungsgedankens anhand von Ausführungsbeispielen und nicht erfindungsgemäßen Alternativen (Figuren 2-5) unter Bezugnahme auf die Zeichnung exemplarisch beschrieben, auf die im Übrigen bezüglich der Offenbarung aller im Text nicht näher erläuterten erfindungsgemäßen Einzelheiten ausdrücklich verwiesen wird. Es zeigen:
- Fig. 1: schematisch die Vermessung der Ausrichtung des Beckens mit zwei Markierungen und einem zum Lot ausgerichteten Kamerasystem,
- Fig. 2: schematisch die Messung der Beweglichkeit einer Hand mit zwei Markierungen,
- Fig. 3: schematisch die Messung der Beweglichkeit eines Arms mit drei Markierungen,
- Fig. 4: schematisch die Messung der Beweglichkeit eines Arms mit vier Markierungen,
- Fig. 5: schematisch die Vermessung der Wirbelsäule mit mehreren Markierungen,
- Fig. 6: schematisch einen Patienten, ein Kamerasystem und vier Markierungen während einer Hüftgelenkoperation.
- Fig. 7: schematisch den messtechnisch relevanten Bereich des optimal ausgerichteten Patienten bei einer Hüftgelenkoperation vor dem Eingriff,
- Fig. 8: schematisch den messtechnisch relevanten Bereich nach gewissen Arbeitsschritten des nicht optimal ausgerichteten Patienten nach einer Hüftgelenkoperation und
- Fig. 9: schematisch den messtechnisch relevanten Bereich nach gewissen Arbeitsschritten des optimal ausgerichteten Patienten bei einer Hüftgelenkoperation mit resultierender Verkürzung der Beinlänge.

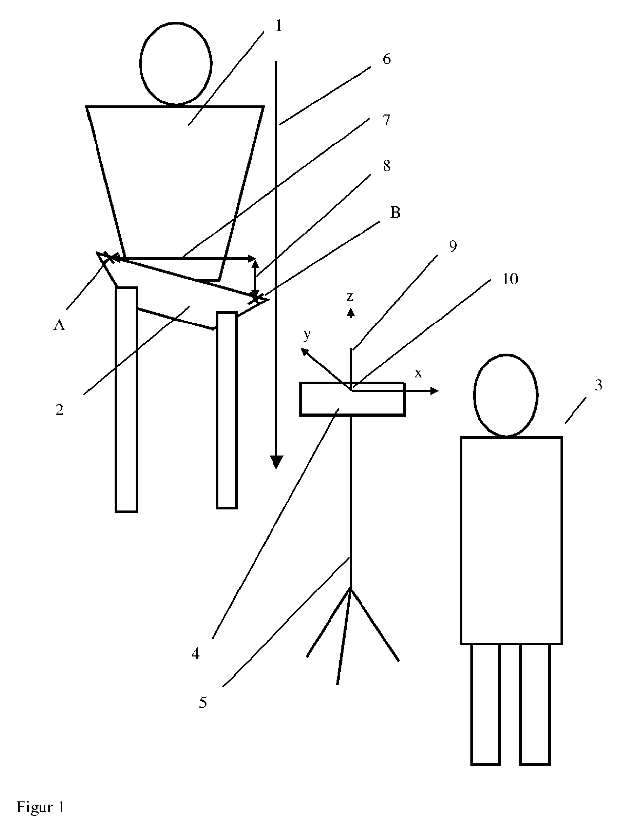

Die Figur 1 zeigt schematisch die Messung der Ausrichtung des Beckens 2 eines aufrecht stehenden Patienten 1. Der Patient 1 hat auf der Haut zwei Markierungen A und B, die vom Anwender 3 unmittelbar in der Nähe von zwei spezifischen Beckenknochen aufgebracht wurden. Das Messsystem 4 auf dem Stativ 5 ist so auf den Patienten 1 ausrichtet, dass beispielsweise die z-Achse des Kamerakoordinatensystems 9 parallel zum Lot 6 ist und der Nullpunkt 10 etwa die gleiche Höhe wie die beiden Markierungen A und B hat.

Der Anwender 3 misst die Raumkoordinaten der Markierungen A und B. Damit berechnet das Messsystem den horizontalen Abstand 7 und die Höhendifferenz 8 der Markierungen A und B. Bei horizontal ausgerichtetem Becken wird die Höhendifferenz 8 gegenüber dem Abstand 7 sehr klein sein. Die Höhendifferenz 8 kann beispielsweise bei ungleich langen Beinen einige Zentimeter betragen.

Die Figur 2 zeigt schematisch die Beweglichkeit einer Hand 11 mit zwei Markierungen A an der Kuppe des Zeigefingers 12 und B an der Kuppe des Daumens 13. Das hier nicht eingezeichnete Messsystem braucht für diese Messung nicht speziell ausgerichtet zu sein, da in diesem Beispiel nur der Abstand 14 zwischen den Markierungen A und B für den Anwender von Interesse ist.

Die Figur 3 zeigt schematisch die Messung der Beweglichkeit eines Arms 20 mit den drei Markierungen A auf dem Oberarm 21 als Kreis, B am Ellbogen 22 und C auf dem Unterarm 23 als Kreuze. Als Mass für die Beweglichkeit dient der Winkel 24 zwischen der Strecke AB 25 und der Strecke BC 26. Das hier nicht eingezeichnete Messsystem braucht für diese Messung beispielsweise nicht auf das Lot ausgerichtet zu sein.

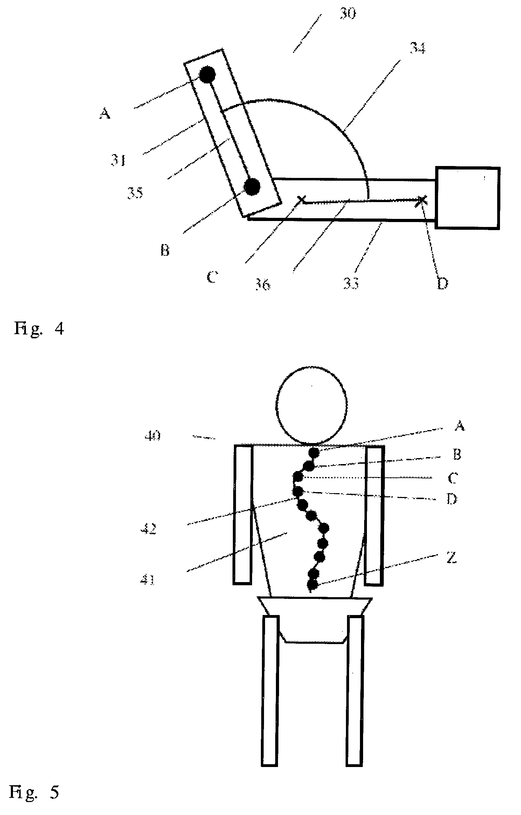

Die Figur 4 zeigt schematisch die Messung der Beweglichkeit eines Arms 30 mit vier Markierungen A und B auf dem Oberarm 31 als Kreise und C und D auf dem Unterarm 33 als Kreuze. Als Maß für die Beweglichkeit dient der Winkel 34 zwischen den beiden Strecken AB 35 und CD 36. Das hier nicht eingezeichnete Messsystem braucht für diese Messung beispielsweise nicht auf das Lot ausgerichtet zu sein.

Die Figur 5 zeigt schematisch bei einem aufrecht stehenden Patienten 40 die Messung der Form der Wirbelsäule 42 mit mehreren Markierungen A, B, C, D, ...Z, die am Rücken 41 auf der Haut beispielsweise unmittelbar über den Dornfortsätzen der Wirbel aufgebracht sind. Das hier nicht eingezeichnete Messsystem kann für diese Messung beispielsweise bezüglich des Lots ausgerichtet sein. Die Koordinaten der gemessenen Markierungen werden dem Anwender aufbereitet zur Verfügung gestellt.

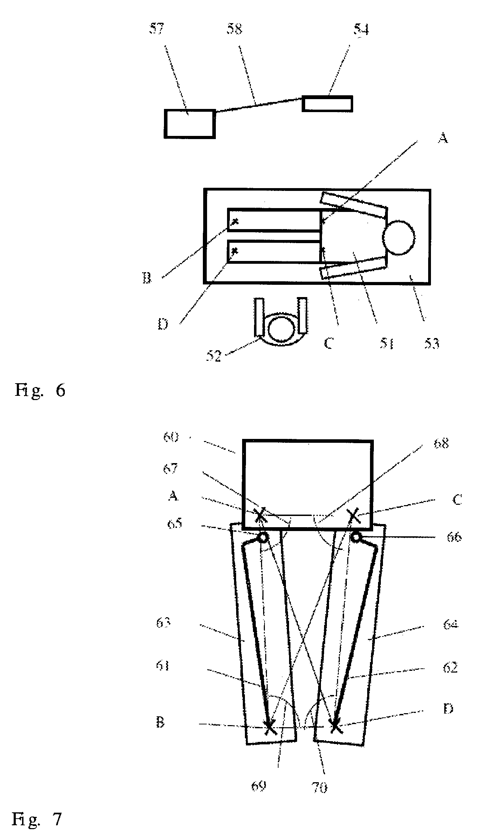

Die Figur 6 zeigt schematisch den Patienten 51, das Messsystem 54, 57, 58 mit vier Markierungen A, B, C, D bei einer Hüftgelenkoperation. Der Patient 51 liegt für den Anwender 52 optimal ausgerichtet auf der OP-Liege 53. Das Kamerasystem 54 befindet sich bei der Messung oberhalb der Markierungen A, B, C und D. Dieses Kamerasystem 54 ist oberhalb der OP-Liege 53 fix angebracht. Die mit dem Kamerasystem 54 erfassten Bilder werden dem Modul 57 mit Recheneinheit und Bildschirm über die Kommunikationsverbindung 58 übermittelt und die Ergebnisse dem Anwender 52 zur Verfügung gestellt.

Eine schematische Darstellung des relevanten Bereichs der OP-Anordnung zeigt Figur 7. Die vier Markierungen A, B, C und D werden vom Anwender vor dem Eingriff auf die Haut des Patienten 60 an für ihn biomechanisch und/oder anatomisch richtigen Stellen beispielsweise mittels Filzstift gezeichnet. Diese Stellen können vom Anwender so ausgewählt werden, dass das erfindungsgemäße Verfahren seine gewohnte Arbeitsweise unterstützt. Die beiden Punkte A und C befinden sich auf der Haut über den vorstehenden Beckenknochen (anterior superior iliac spine). Die beiden Punkte B und D sind auf Hautstellen nahe geeigneten Teilen der Fußgelenke gezeichnet. Die Punkte 65 und 66 sind die Drehzentren der Hüftgelenke. Der Drehpunkt 65 befindet sich typischerweise nahe der Strecke AB. Der Drehpunkt 66 befindet sich typischerweise nahe der Strecke CD. Die rechten und linken Beinknochen (Femur, Tibia, Fibula) 61 und 62 sowie das rechte Bein 63 und das linke Bein 64 vom Patienten sind schematisch eingezeichnet.

Aus der für den Anwender optimalen biomechanischen Ausrichtung des Patienten und den Positionen der Markierungen resultiert eine Spiegelsymmetrie, die bei der Analyse der Messungen nützlich und zweckmäßig ist.

Folgende Beziehungen zwischen Strecken und Winkeln können damit ermittelt werden: Alle vier Markierungen A, B, C und D liegen typischerweise ungefähr auf der gleichen Ebene. Die Strecke BD ist etwas kürzer als die Strecke AC, falls beide Füße nahe beieinander liegen. Die Strecken AC und BD sind zueinander ungefähr parallel. Die diagonalen Strecken AD und BC sind ungefähr gleich lang. Der Winkel 67 zwischen den Strecken AB und AC ist ungefähr gleich dem Winkel 68 zwischen den Strecken AB und BD. Der Winkel 69 zwischen den Strecken AB und BD ist ungefähr gleich dem Winkel 70 zwischen den Strecken CD und BD. Als Beinlängen werden für dieses Beispiel die Strecken AB und CD definiert.

Damit sind die in der Figur 7 eingezeichneten bei der ersten Messung ermittelten sechs Strecken AB, AC, AD, CD, CB und BD und vier Winkel 67, 68, 69, 70 für die Ausgangslage bekannt.

Eine durch den Eingriff resultierende Beinlängenänderung kann durch Vergleich der nach dem Eingriff durchgeführten zweiten Messung mit der ersten Messung unter Verwendung der sechs Strecken AB, AC, AD, CD, CB und BD und der vier Winkel 67, 68, 69, 70 mathematisch beschrieben werden.

Wird beispielsweise die linke Hüfte operiert, kann sich die Länge des Vektors CD bezüglich der ersten Messung ändern. Die Beinlängenänderung ist im Wesentlichen die Differenz der Strecken CD vor dem Eingriff und der Strecke CD nach dem Eingriff. Des Weiteren zeigen die beiden Diagonalen AD und CB und die beiden Winkel 67 und 68 wie der Patient bei den Messungen auf der OP-Liege ausgerichtet wurde. Dabei muss der Patient nicht an derselben Stelle im Raum und in derselben Lage sein bezüglich des Kamerasystems wie bei der ersten Messung. Für die Messung nach dem Eingriff wird angestrebt, dass der Patient ungefähr die gleiche optimale biomechanische Ausrichtung einnimmt wie bei der ersten Messung. Damit kann die Änderung der Beinlänge durch den Eingriff berechnet werden. Kleinere Abweichungen der biomechanischen Patientenausrichtung bei der zweiten Messung bezüglich der ersten Messung werden durch die Analyse erkannt und bei der Berechnung der Beinlängenänderung berücksichtigt.

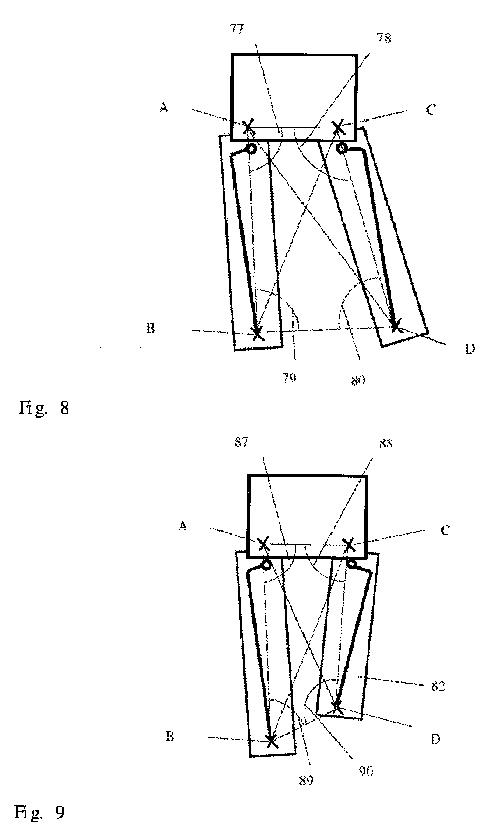

Die Figur 8 zeigt schematisch die Lage der Markierungen bei einem nicht optimal ausgerichteten Patienten nach dem Eingriff ohne Änderung der linken Beinlänge. Die nicht optimale Ausrichtung des Patienten manifestiert sich beispielsweise durch die nicht gleich langen diagonalen Strecken AD und BC, durch die geänderte Länge der Strecke BD und durch die geänderten Winkel 77, 78, 79, 80. Der Anwender kann mit Hilfe dieser Angaben den Patienten für die Messung und Beurteilung der Qualität des Eingriffs optimal ausrichten.

Die Figur 9 zeigt schematisch die Lage der Markierungen bei einem optimal ausgerichteten Patienten jedoch mit einer durch den Eingriff verursachten Verkürzung der Länge des Beins 82. Das nicht optimale Ergebnis des Eingriffs manifestiert sich durch die nicht gleichen diagonalen Strecken AD und BC, durch die geänderte Länge der Strecke BD und durch die geänderten Winkel 88, 89, 90. Der Anwender kann mit Hilfe dieser Angaben den weiteren Verlauf des Eingriffs dieser Situation anpassen und die nötigen Korrekturen anbringen.

Am Ende des Eingriffs kann der Patient ein letztes Mal vermessen werden. Diese Messung dient für den Anwender als Qualitätskontrolle für den Eingriff.

Die erfassten Bilder und Analyseprotokolle können für spätere Verwendungen archiviert werden.

Die Erfindung eignet sich in ihrer Anwendung nicht nur für Hüftgelenksoperationen. Überall wo eine biomechanische Änderung durch eine medizinische Behandlung möglich ist, kann dies mit dem beschriebenen Verfahren messtechnisch erfasst werden und die Ergebnisse der Analysen können dem Anwender zur Verfügung gestellt werden.

Die Vermessung beinhaltet eine Stereoaufnahme oder eine Serie von Stereoaufnahmen, bei der der Anwender den Patienten in geeigneter Weise behandelt, bewegt oder umlagert oder der Patient bestimmte Körperteile bewegen muss.

Eine weitere Anwendung der Erfindung ist beispielsweise während einem Heilungsverlauf die Kontrolle und /oder Modifikation für die Nachbehandlung.

## Patentansprüche

1. Verfahren zum Betreiben eines optischen, stereometrischen Mess-Systems zur Ermittlung einer Beinlängenänderung, bei dem auf der Haut eines ausgerichteten Patienten Markierungen über den beiden vorstehenden Beckenknochen (anterior superior iliac spine) und an den beiden Fußgelenken angebracht und bei dem für eine erste und eine zweite Messung am ausgerichteten Patienten mit mindestens zwei ausgerichteten Kameras die Raumkoordinaten dieser Markierungen sowie deren Beziehungen untereinander in Form von Strecken und Winkeln ermittelt werden, ***dadurch gekennzeichnet, dass*** die Ermittlung der Beinlängenänderung den Vergleich der Strecken und Winkel sowie die Spiegelsymmetrie der Positionen der Markierungen beinhaltet.

2. Verfahren nach Anspruch 1, ***dadurch gekennzeichnet, dass*** durch Vergleich mit einem von den Kameras erfassten vorbekannten Maß, eine absolute Länge bestimmt wird.

3. Verfahren nach einem der vorhergehenden Ansprüche, ***dadurch gekennzeichnet, dass*** die Kameras stationär im Raum angeordnet sind.

4. Verfahren nach einem der Ansprüche 1 oder 2, ***dadurch gekennzeichnet, dass*** die Kameras fest miteinander verbunden aber mobil im Raum angeordnet sind.

## Claims

1. Method of operating an optical, stereometric measuring system for determining a change of length of a leg, in which on the skin of a positioned patient markings are applied on the two projecting pelvic bones (anterior superior iliac spine) and on both ankles and in which for a first and a second measurement on the positioned patient with at least two orientated cameras the spatial coordinates of these marking as well as their relationships to each other are determined in the form of distances and angles, ***characterised in that*** the determination of the change in the length of a leg includes the comparison of the distances and angles as well as the mirror symmetry of the positions of the markings.

2. Method according to claim 1. ***characterised in that*** through comparison with a previously known measurement recorded by the cameras an absolute length is determined.

3. Method according any one of the preceding claims, ***characterised in that*** the cameras are arranged in a stationary manner in the room.

4. Method according to any one of claim 1 or 2, ***characterised in that*** the cameras are firmly connected to each other but are arranged in a mobile manner in the room.

## Revendications

1. Procédé destiné à faire fonctionner un système de mesure optique. stéréométrique pour déterminer la variation de longueur d'une jambe, lors duquel on applique sur la peau d'un patient aligné des marquages sur les deux os iliaques saillants (anterior superior iliac spine) et sur les deux chevilles et lors duquel, pour une première et une deuxième mesures sur le patient aligné, on détermine à l'aide d'au moins deux caméras alignées les coordonnées spatiales desdits marquages, ainsi que leurs relations mutuelles sous la forme de trajets et d'angles, ***caractérisé en ce que*** la détermination de la variation de la longueur de jambe contient la comparaison des trajets et angles, ainsi que la symétrie spéculaire des positions des marquages.

2. Procédé selon la revendication 1, ***caractérisé en ce que*** par comparaison avec une dimension précédemment connue, détectée par la caméra, on définit une longueur absolue.

3. Procédé selon l'une quelconque des revendications précédentes, ***caractérisé en ce que*** les caméras sont placées de manière stationnaire dans l'espace.

4. Procédé selon l'une quelconque des revendications 1 ou 2, ***caractérisé en ce que*** les caméras sont placées en étant fixement reliées l'une à l'autre, mais mobiles dans l'espace.
